# EUROPEAN PATENT SPECIFICATION

(11) **EP 2 564 899 B1**
(45) Date of publication and mention of the grant of the patent: **24.12.2014**
(21) Application number: 12180093.2
(22) Date of filing: 10.08.2012
(51) Int. Cl.: A61N 7/02, G10K 11/34, A61N 7/00, A61B 8/08

(54) **Medical apparatus**
Medizingerät
Appareil médical

(30) Priority: 05.09.2011 KR 20110089865
(43) Date of publication of application: 06.03.2013
(73) Proprietor: Samsung Electronics Co., Ltd., Suwon-si, Gyeonggi-do, 443-742 (KR)
(72) Inventor: Lee, Ho-taik, 446-712 Gyeonggi-do (KR); Lee, Hyoung-ki, 446-712 Gyeonggi-do (KR); Bang, Won-chul, 446-712 Gyeonggi-do (KR); Shim, Mun-bo, 446-712 Gyeonggi-do (KR); Park, Jun-ho, 446-712 Gyeonggi-do (KR)
(74) Representative: Grünecker, Kinkeldey, Stockmair & Schwanhäusser

(56) References cited:
- EP-A1- 1 936 404
- WO-A2-2011/024074
- US-A1- 2010 030 076

## Description

### BACKGROUND

### 1. Field

The following description relates to a medical apparatus.

### 2. Description of Related Art

As medical science has developed, non-invasive forms of surgery have arisen to treat a lesion tissue of the human body, such as, for example, a tumor, without use of a scalpel or a needle. For example, high intensity focused ultrasound (HIFU) treatment is a form of non-invasive surgery that uses ultrasound. The ultrasound used in the HIFU treatment is not harmful to the human body. The HIFU treatment has become widely used to treat the lesion tissue without use of a scalpel or a needle.

An intensity of the ultrasound used in the HIFU treatment is about a hundred thousand times greater than an intensity of ultrasound used for diagnosis. The ultrasound of the HIFU treatment is focused on and irradiated onto a lesion part to be treated, thereby causing focal destruction or necrosis of the lesion tissue. That is, energy of the ultrasound is focused on and irradiated onto a part of the lesion tissue of the human body and is transformed into thermal energy, thereby causing a temperature of the irradiated part to rise and resulting in coaguable necrosis of the lesion tissue. In this case, since the temperature of the irradiated part rises instantaneously, heat may be prevented from being dispersed into a periphery of the irradiated part. As a result, only the irradiated part of the lesion tissue may be efficiently removed.

An apparatus utilizing the HIFU treatment uses a transducer that generates and irradiates ultrasound for treatment in response to an electrical signal. For example, a multielement ultrasound transducer may be used to achieve the effect of the HIFU treatment. In order to respectively apply each of a plurality of input signals to multiple elements of the multielement ultrasound transducer of an ultrasound apparatus, a unit generating the input signals should have the same number of output channels as the number of elements of the multielement ultrasound transducer. Therefore, as ultrasound transducer elements are added to a HIFU treatment system, a method by which a signal of each transducer element may be controlled is subject to increased complexity.

### SUMMARY

In one general aspect, a medical apparatus includes an ultrasound transducer unit including a plurality of ultrasound transducer elements, the ultrasound transducer elements being configured to transform signals into ultrasound and output the transformed ultrasound, a grouping unit configured to group the ultrasound transducer elements into a plurality of groups, and a signal generating unit configured to generate the signals to be transformed into the ultrasound and output the generated signals to the groups via a plurality of output channels respectively corresponding to the groups.

The medical apparatus may include that the signal generating unit is further configured to output one or more common signals of the signals to be transformed into the ultrasound via one or more of the output channels, respectively, to respective ones of the groups comprising two or more of the ultrasound transducer elements.

The medical apparatus may include a channel selection unit configured to select two or more of the output channels via which the signals to be transformed into the ultrasound are to be output to the groups.

The medical apparatus may include a user interface configured to receive grouping information specifying a quantity of the groups in which the ultrasound transducer elements are to be grouped. The channel selection unit is further configured to select the two or more of the output channels according to the received grouping information.

The medical apparatus may include that the grouping unit is further configured to group the ultrasound transducer elements into activated ultrasound transducer elements and deactivated ultrasound transducer elements, and group the activated ultrasound transducer elements into two or more groups.

The medical apparatus may include a user interface configured to receive grouping information specifying a quantity of the activated ultrasound transducer elements to be input. The grouping unit is further configured to group the activated ultrasound transducer elements and deactivated ultrasound transducer elements according to the received grouping information.

The medical apparatus includes that the grouping unit is further configured to sample the activated ultrasound transducer elements and the deactivated ultrasound transducer elements via random extraction.

The medical apparatus may include that the grouping unit is further configured to group adjacent ones of the ultrasound transducer elements into a single group.

The medical apparatus may include a phase modulator configured to modulate phase of the signals to be outputted to the groups to form a focus of the ultrasound transducer unit in a target region onto which the ultrasound is to be irradiated, and a signal amplifier configured to amplify amplitudes of the phase modulated signals.

The medical apparatus may include a processor configured to calculate phases and amplification gains of the signals to be outputted to the groups to control the phase modulator and the signal amplifier, the phases and the amplification gains being calculated according to grouping information transmitted from the grouping unit.

The medical apparatus may include that the processor is further configured to control the phase modulator and the signal amplifier to form a multi-focus from the ultrasound irradiated by the ultrasound transducer unit in response to the signals outputted to the groups.

In yet another general aspect, there is provided a non-transitory computer readable recording medium having recorded thereon a program for executing the method of controlling the medical apparatus.

Other features and aspects may be apparent from the following detailed description, the drawings, and the claims. The invention is defined in the claims. Other embodiments are described for illustrative purposes only.

### BRIEF DESCRIPTION OF THE DRAWINGS

FIG. 1 is a diagram illustrating an example of a medical apparatus in use.
FIG. 2 is a diagram illustrating an example of an ultrasound transducer unit of a medical apparatus.
FIG. 3 is a block diagram illustrating an example of a medical apparatus.
FIG. 4 is a block diagram illustrating another example of a medical apparatus.
FIG. 5 is a block diagram illustrating an example of a grouping unit of a medical apparatus.
FIG. 6A and 6B are block diagrams illustrating an example of adjacent ultrasound transducer elements grouped in an ultrasound transducer unit having an array of ultrasound transducer elements arranged in the form of a ring.
FIG. 7 is a block diagram illustrating yet another example of a medical apparatus.
FIG. 8 is a block diagram illustrating still another example of a medical apparatus in a diagnosis device used in diagnosing a lesion tissue of the human body.
FIG. 9 is a flowchart illustrating an example of a method of controlling a medical apparatus.
FIG. 10 is a flowchart illustrating an example of a grouping of a plurality of ultrasound transducer elements in a method of controlling a medical apparatus.
FIG. 11 is a flowchart illustrating an example of a generation of signals to be input according to groups in a method of controlling a medical apparatus.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following detailed description is provided to assist the reader in gaining a comprehensive understanding of the methods, apparatuses, and/or systems described herein. Accordingly, various changes, modifications, and equivalents of the systems, apparatuses and/or methods described herein will be suggested to those of ordinary skill in the art. In addition, descriptions of well-known functions and constructions may be omitted for increased clarity and conciseness.

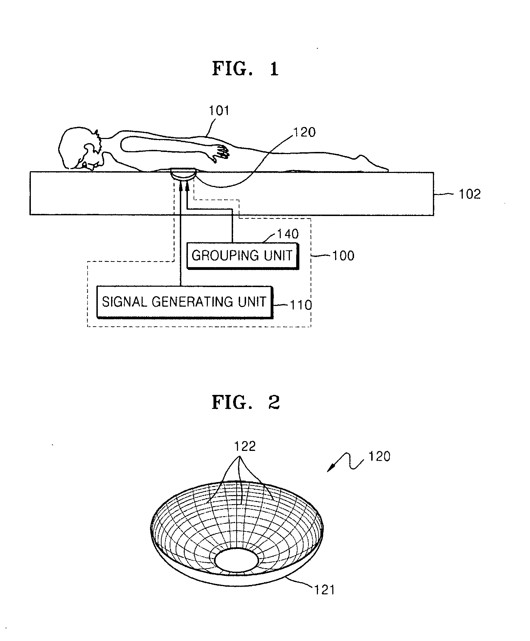

FIG. 1 is a diagram illustrating an example of a medical apparatus 100 in use. Referring to the example of FIG. 1, the medical apparatus 100 includes a signal generating unit 110, an ultrasound transducer unit 120, and a grouping unit 140. It will be understood by one of ordinary skill in the art that other general elements than the elements illustrated in FIG. 1 may be further included in the medical apparatus 100.

In this example, the ultrasound transducer unit 120 is installed in a bed 102 on which a person 101 to be inspected is to lie. When the signal generating unit 110 outputs a signal to the ultrasound transducer unit 120, the ultrasound transducer unit 120 generates ultrasound from the outputted signal and irradiates the ultrasound onto a particular part of the person 101 requiring treatment or diagnosis. An example of the outputted signal is an electrical signal. However, this is only an example, and any other signal known to one of ordinary skill in the art may be used.

FIG. 2 is a diagram illustrating an example the ultrasound transducer unit 120 of the medical apparatus 100. The example of the ultrasound transducer unit 120 illustrated in FIG. 2 is a concave type of focused ultrasonic transducer unit. In an operation example, focal points of ultrasound transducer elements 122 of the ultrasound transducer unit 120 are created corresponding to a region onto which ultrasound is to be irradiated. An example of the region onto which the ultrasound is to be irradiated is part of the breast, the liver, the abdomen, or any other organ or part of the human body known to one of ordinary skill in the art. In addition, the ultrasound transducer unit 120 in the example of FIG. 2 is only an example. Other units known to one of ordinary skill in the art to convert signals into ultrasound may be used.

Referring to the example of FIG. 2, the ultrasound transducer unit 120 includes a plurality of ultrasound transducer elements 122 disposed on a disc-shaped support plate 121 having a concave middle part. Each of the ultrasound transducer elements 122 in this example modulates an electrical signal, which is outputted from the signal generating unit 110 and has a predetermined amplitude and phase, into ultrasound having a predetermined intensity and phase. The ultrasound transducer elements 122 in this example may be manufactured as piezoelectric transducers or any other elements known to one of ordinary skill in the art to modulate an electrical signal into ultrasound.

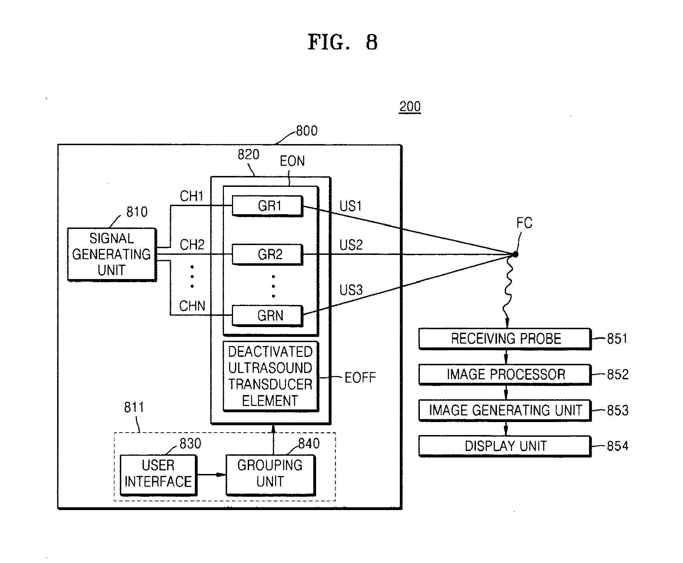

Each ultrasound transducer element 122 of this example is focuses the generated ultrasound on a particular part of the body of the person 101 to be inspected requiring treatment or diagnosis. In this example, the ultrasound focused on the part to be irradiated is transformed into thermal energy, thereby causing a temperature of the irradiated part to rise and resulting in focal destruction or necrosis of a lesion tissue. In an example described later with reference to FIG. 8, ultrasound focused on the part to be irradiated and being partially reflected from tissue layers is transformed into electrical pulses using a probe, thereby resulting in the capture of an image of an inner tissue of the body.

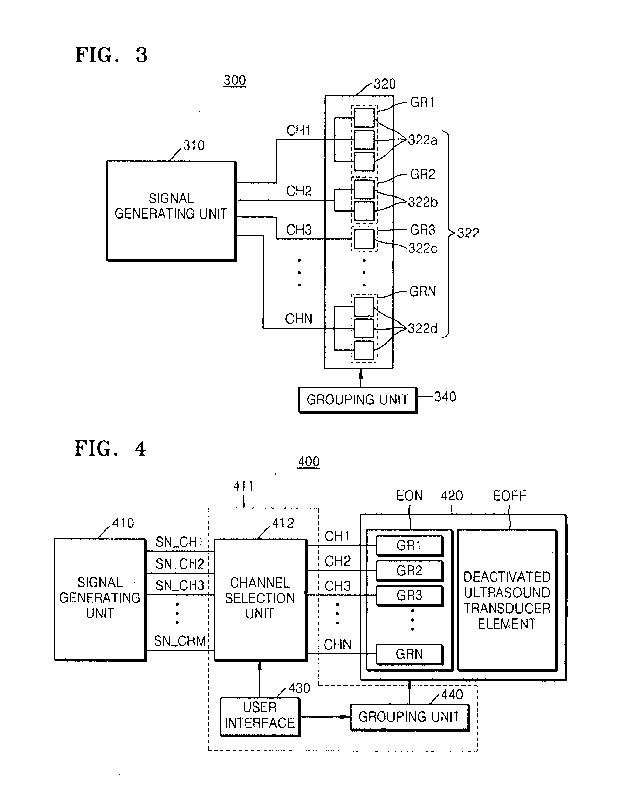

FIG. 3 is a block diagram illustrating an example of a medical apparatus 300. Referring to the example of FIG. 3, the medical apparatus 300 includes a signal generating unit 310, an ultrasound transducer unit 320, and a grouping unit 340. It will be understood by one of ordinary skill in the art that other general elements than the elements illustrated in FIG. 3 may be further included in the medical apparatus 300. For example, a device configured to match impedances of the ultrasound transducer elements 322 of the ultrasound transducer unit 320 may be disposed between the signal generating unit 310 and the ultrasound transducer unit 320.

In the example of FIG. 3, the signal generating unit 310 outputs electrical signals to the ultrasound transducer unit 320 via output channels CH1 to CHN. In this example, one or more of the output channels CH1 to CHN of the signal generating unit 310 respectively outputs a common signal to two or more of the ultrasound transducer elements 322.

The ultrasound transducer elements 322 in this example are grouped into a plurality of groups GR1 to GRN of the ultrasound transducer unit 320. Each of the output channels CH1 to CHN of the signal generating unit 310 respectively outputs an electrical signal according to the plurality of groups GR1 to GRN. In the example of FIG. 3, output channel CH1 outputs a common signal to a group GR1 of ultrasound transducer elements 322a, output channel CH2 outputs a common signal to a group GR2 of ultrasound transducer elements 322b, output channel CH3 outputs a common signal to a group GR3 containing an ultrasound transducer element 322c, and output channel CHN outputs a common signal to a group GRN of ultrasound transducer elements 322d.

In this example, the grouping unit 140 groups ultrasound transducer elements 322 into two or more of the groups GR1 to GRN. Further, one or more of the groups GR1 to GRN includes two or more ultrasound transducer elements 322.

FIG. 4 is a block diagram illustrating an example of a medical apparatus 400. Referring to the example illustrated in FIG. 4, the medical apparatus 400 includes a signal generating unit 410, an ultrasound transducer unit 420, and a control unit 411 including a channel selection unit 412, a user interface 430 and a grouping unit 440. Descriptions of the signal generating unit 410 and the ultrasound transducer unit 420 of FIG. 4 that would have been repeated from descriptions of the signal generating unit 310 and the ultrasound transducer unit 320 of FIG. 3 are omitted.

The user interface 430 of the medical apparatus 400 allows a user to input instructions or information relating to ultrasound treatment therein. In this example, the user is a medical expert or any other entity known to one of ordinary skill in the art to need to input instructions or information relating to ultrasound treatment in a medical apparatus. The user may input grouping information, which includes the number of groups N of the ultrasound transducer elements to the user interface 430. The user interface 430 may be implemented with an input device, such as a keyboard, a mouse, or any other input device known to one of ordinary skill in the art, or a graphical user interface (GUI).

In the example illustrated in FIG. 4, the signal generating unit 410 generates signals and outputs the signals outputs via a plurality of M channels SN_CH1 to SN_CHM. The channel selection unit 412 selects a plurality of output channels CH1 to CHN to output electrical signals according to groups of the ultrasound transducer unit 420 from among the plurality of M channels SN_CH1 to SN_CHM of the signal generating unit 410.

The channel selection unit 412 outputs electrical signals via output channels CH1 to CHN according to groups made by the grouping unit 440. In this example, the number of output channels of the channel selection unit 412 are equal to the number of groups N of the ultrasound transducer unit 420. The number of groups N is less than the number of ultrasound transducer elements.

In addition, in the example of FIG. 4, the user inputs grouping information including the number of activated ultrasound transducer elements together with the number of groups N of the ultrasound transducer elements to the user interface 430. In this case, the grouping unit 440 groups the ultrasound transducer elements into activated ultrasound transducer elements EON and deactivated ultrasound transducer elements EOFF in consideration of the number of activated ultrasound transducer elements input by the user. The grouping unit 440 groups the activated ultrasound transducer elements EON into groups GR1 to GRN. The activated ultrasound transducer elements EON generate ultrasound in response to electrical signals according to groups GR1 to GRN. The deactivated ultrasound transducer elements EOFF are not connected to the output channels CH1 to CHN of the channel selection unit 412 and, as such, do not generate ultrasound.

When the user inputs the number of groups N of the ultrasound transducer unit 420 to the user interface 430, information regarding the input number of groups N is outputted to the channel selection unit 412. Then, the channel selection unit 412 selects the output channels CH1 to CHN corresponding to the information regarding the input number of groups N from among the plurality of M channels SN_CH1 to SN_CHM. The channel selection unit 412 in this example may be implemented with a switching circuit to select N output channels from among M channels output by the signal generating unit 410.

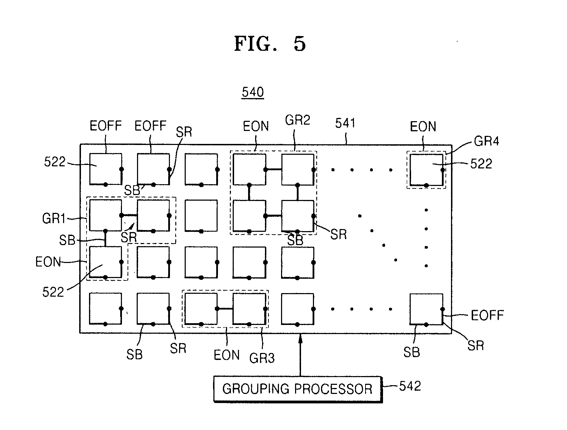

FIG. 5 is a block diagram illustrating an example of a grouping unit 540 of a medical apparatus. The grouping unit 540 of this example allows the ultrasound transducer elements 522 to be connected to each other according to grouping information of the ultrasound transducer elements 522 transmitted from a user interface. To this end, the grouping unit 540 includes a substrate 541, switches SR and SB implemented on the substrate 541, and a grouping processor 542.

Here, each of the ultrasound transducer elements 522 includes a switch SR switched to electrically short or isolate the ultrasound transducer elements 522 from right adjacent ultrasound transducer elements 522. In addition, each of the ultrasound transducer elements 522 in FIG. 5 includes a switch SB switched to electrically short or isolate the ultrasound transducer elements 522 from downward adjacent ultrasound transducer elements 522.

The grouping processor 542 groups the ultrasound transducer elements 522 into activated ultrasound transducer elements EON and deactivated ultrasound transducer elements EOFF. In addition, the grouping processor 542 controls the switches SR and SB of the ultrasound transducer elements 522 to group the ultrasound transducer elements 522 into groups GR1 to GR4.

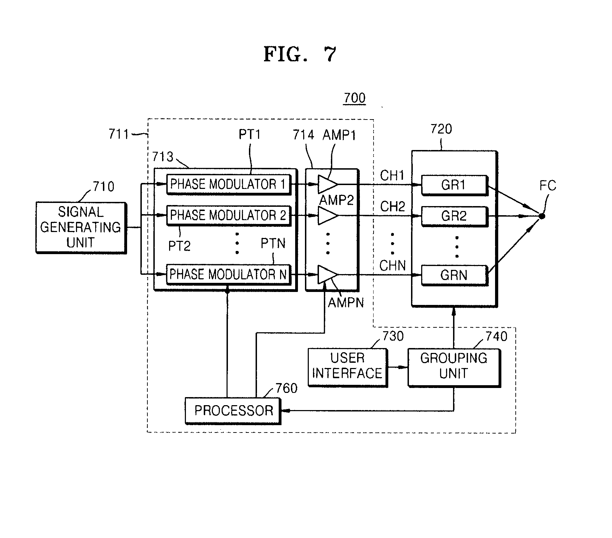

FIG. 7 is a block diagram illustrating an example of a medical apparatus 700. Referring to the example illustrated in FIG. 7, the medical apparatus 700 includes a control unit 711 including a phase modulator 713, a signal amplifier 714, a user interface 730, a grouping unit 740, and a processor 760. Here, different amplitudes and phases may be applied to electrical signals to be input to groups GR1 to GRN of a plurality of ultrasound transducer elements to focus ultrasound irradiated by the plurality of ultrasound transducer elements on a focus FC. Descriptions of the signal generating unit 710, the ultrasound transducer unit 720, the user interface 730, and the grouping unit 740 of FIG. 7 that would have been repeated from descriptions of elements in FIGS. 3 and 4 are omitted.

In this example, the signal generating unit 710 generates electrical signals. The generated electrical signals are input to phase modulator elements PT1 to PTN of the phase modulator 713. The phase modulator elements PT1 to PTN modulate phases of the electrical signals input by the signal generating unit 710 and output the electrical signals having the modulated phases to amplifier elements AMP1 to AMPN of the signal amplifier 714.

In order to compensate for a difference in distances between the ultrasound transducer elements that belong to different groups GR1 to GRN and the focus FC, the phase modulator elements PT1 to PTN of the phase modulator 713 outputs electrical signals having different phases so that ultrasound signals generated from the ultrasound transducer elements that belong to different groups GR1 to GRN have different transmission delay times. To this end, the grouping unit 740 transmits grouping information to the processor 760 to control the phase modulator 713 by analyzing the transmitted grouping information and calculating phases of electrical signals to be modulated by the phase modulator elements PT1 to PTN.

The amplifier elements AMP1 to AMPN of the signal amplifier 714 amplifies the electrical signals input from the phase modulator elements PT1 to PTN with appropriate gains to output the amplified electrical signals via output channels CH1 to CHN. To this end, the grouping unit 740 transmits the grouping information to the processor 160 to control the signal amplifier 714 by analyzing the transmitted grouping information and calculating gains of electrical signals to be amplified by the amplifier elements AMP1 to AMPN.

Thus, the control unit 711 controls the ultrasound transducer elements to generate ultrasound having different phases and intensities according to groups GR1 to GRN in response to electrical signals having different phases and amplitudes via the channels CH1 to CHN. The control unit 711 controls the ultrasound to have the same phase and intensity when ultrasound irradiated according to groups GR1 to GRN reaches the focus FC on which the ultrasound is to be focused. In addition, the control unit 711 controls the phases and amplitudes of the electrical signals input to the groups GR1 to GRN of the ultrasound transducer unit 720 to vary the position of the focus FC on which the ultrasound is to be focused within a predetermined target region that requires treatment or diagnosis, i.e., a region onto which the ultrasound is to be irradiated.

Moreover, the control unit 711 controls the ultrasound transducer unit 720 to make a multi-focus using the ultrasound generated from the ultrasound transducer unit 720. For example, in order to make a double focus (first and second focuses), the processor 760 groups the groups GR1 to GRN of the ultrasound transducer unit 720 into first groups and second groups. In this case, the processor 760 controls the phase modulator elements PT1 to PTN of the phase modulator 713 and the amplifier elements AMP1 to AMPN of the signal amplifier 714 relating to the electrical signals applied to the first groups via output channels to irradiate the ultrasound therefrom onto a first focal position by using the ultrasound transducer elements of the first groups. The processor 760 controls the phase modulator elements PT1 to PTN of the phase modulator 713 and the amplifier elements AMP1 to AMPN of the signal amplifier 714 relating to the electrical signals applied to the second groups via output channels to irradiate the ultrasound therefrom onto a second focal position by using the ultrasound transducer elements of the second groups.

FIG. 8 is a block diagram illustrating an example of a medical apparatus 800 in a diagnosis device 200 used in diagnosing a lesion tissue of the human body Referring to the example illustrated in FIG. 8, a diagnosis device 200 includes the medical apparatus 800, a receiving probe 851, an image processor 852, an image generating unit 853, and a display unit 854. The medical apparatus 800 includes a signal generating unit 810, an ultrasound transducer unit 820, a user interface 830, and a grouping unit 840. Descriptions of the medical apparatus 800, the signal generating unit 810, the ultrasound transducer unit 820, the user interface 830, and the grouping unit 840 of FIG. 8 that would have been repeated from descriptions of elements in FIGS. 1, 3, 4, and 7 are omitted.

The receiving probe 851 of this example may be manufactured as a piezoelectric transducer or any other elements known to one of ordinary skill in the art to modulate ultrasound into an electrical signal. When ultrasound is transferred by the medical apparatus 800 to a particular part of the body, the ultrasound is partially reflected from or diffracted at layers between different tissues. For example, the ultrasound is reflected from a place of the body where densities of blood cells in blood plasma, structures in organs, or any other organs or tissues known to one of ordinary skill in the art vary or are diffracted at a space between the structures. When an HIFU transducer unit is used, the ultrasound transducer unit 820 generates ultrasonic signals US1 to USN according to groups GR1 to GRN, and the ultrasonic signals US1 to USN irradiated by the ultrasound transducer unit 120 are concentratively reflected from or diffracted at the focus FC. In this way, ultrasound irradiated by a plurality of ultrasound transducer elements is transmitted to the focus FC and are collectively reflected from or diffracted at the focus FC.

The ultrasonic signals US1 to USN reflected from the body vibrate the receiving probe 851. The receiving probe 851 outputs electrical pulses having predetermined amplitudes and phases according to the vibrations. The image processor 852 performs image processing on the electrical pulses output from the receiving probe 851 to generate image data. The image generating unit 853 generates ultrasound images based on the image data. For example, the image generating unit 853 transforms the image data according to a format of the display unit 854 to generate ultrasound images. In addition, the image generating unit 853 transmits the generated ultrasound images to the display unit 854. The ultrasound images generated by the image generating unit 853 are displayed in graphics on the display unit 854. An example of the display unit 854 may be a liquid crystal display (LCD) screen or any device known to one of ordinary skill in the art to display ultrasound images on a screen, paper, or space.

The receiving probe 851 may include one or more transducer elements. The one or more transducer elements of the receiving probe 851 may be a piezoelectric transducer separated from the ultrasound transducer unit 820 or any other elements known to one of ordinary skill in the art to modulate ultrasound into an electrical signal that is separated from the ultrasound transducer unit 820. The receiving probe 851 may include one or more ultrasound transducer elements of the ultrasound transducer unit 820.

When one or more of the ultrasound transducer elements of the ultrasound transducer unit 820 is used as the receiving probe 851, the ultrasound transducer elements that constitute the receiving probe 851 are not limited to performing of a function of receiving ultrasound signals reflected from the body and may simultaneously perform a function of irradiating the ultrasound signals onto the body or any other function known to one of ordinary skill in the art that is able to be performed by ultrasound transducer elements.

The ultrasound images generated by the ultrasound imaging device may be a variety of images in a B-mode, a Doppler mode, or any other mode known to one of ordinary skill in the art in which an ultrasound image may be generated. In addition, the ultrasound images may have a two-dimensional or three-dimensional shape. Three-dimensional ultrasound images are appropriate to provide stereoscopic ultrasound images by using x-, y-, and z-axes. The three-dimensional ultrasound images may be generated by using three-dimensional receiving signals input to a plurality of transducers or a combination of a plurality of two-dimensional image data.

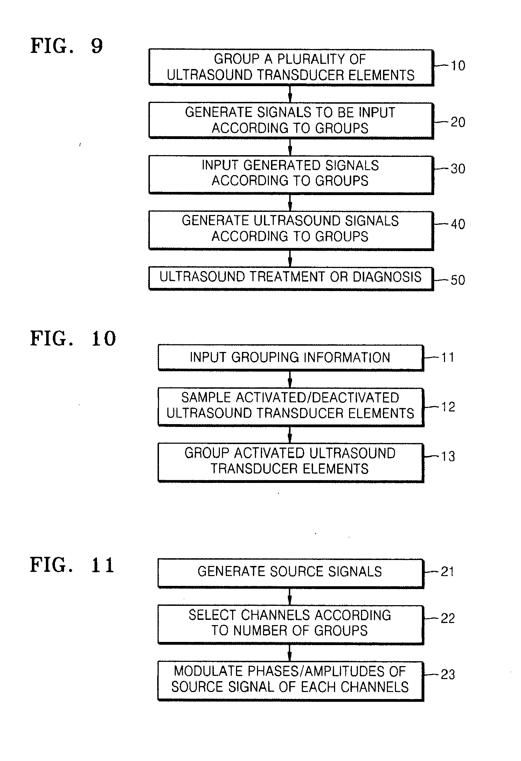

FIG. 9 is a flowchart illustrating an example of a method of controlling a medical apparatus. In this example, the method of controlling a medical apparatus illustrated in FIG. 9 may be implemented by the medical apparatuses 100, 300, 400, 700, and 800 of FIGS. 1, 3, 4, 7, and 8, respectively. Although omitted below unless otherwise specified, descriptions of the medical apparatuses 100, 300, 400, 700, and 800 of FIGS. 1, 3, 4, 7, and 8, respectively, may also be applied to the example of the method of controlling a medical apparatus illustrated in FIG. 9.

Referring to the example illustrated in FIG. 9, a plurality of ultrasound transducer elements of an ultrasound transducer unit are grouped (10) into a plurality of groups.

FIG. 10 is a flowchart illustrating an example of a grouping of a plurality of ultrasound transducer elements in a method of controlling a medical apparatus. Referring to an example illustrated in FIG 10, a user inputs (11) grouping information to a user interface. In this example, the user is a medical expert or any other entity known to one of ordinary skill in the art to need to input instructions or information relating to ultrasound treatment in a medical apparatus. In this example, the grouping information may include the number of groups of a plurality of ultrasound transducer elements, the number of activated ultrasound transducer elements, or any other information known to one of ordinary skill in the art to assist in grouping ultrasound transducer elements. Here, when the plurality of ultrasound transducer elements is n, a number of groups N is equal to or greater than 2 and equal to or less than (n-1), and a number k of activated ultrasound transducer elements is equal to or greater than (N+1) and equal to or less than n. This is because two or more ultrasound transducer elements may be included in one or more of the number of groups N.

Activated ultrasound transducer elements and deactivated ultrasound transducer elements are sampled (12) from among the ultrasound transducer elements. When grouping information k = n is input, all ultrasound transducer elements are sampled as activated ultrasound transducer elements and the sampling of the ultrasound transducer elements and deactivated ultrasound transducer elements may be omitted. If only information regarding the number of groups N and the number k of activated ultrasound transducer elements is input, the k activated ultrasound transducer elements are extracted randomly from n ultrasound transducer elements.

The activated ultrasound transducer elements are grouped (13) into the inputted number of groups N of the grouping information. If the grouping information regarding all ultrasound transducer elements is input, the number of groups N is grouped according to the input grouping information. If only information regarding the number of groups N and the number k of activated ultrasound transducer elements is input, the k activated ultrasound transducer elements are grouped randomly into the number of groups N groups.

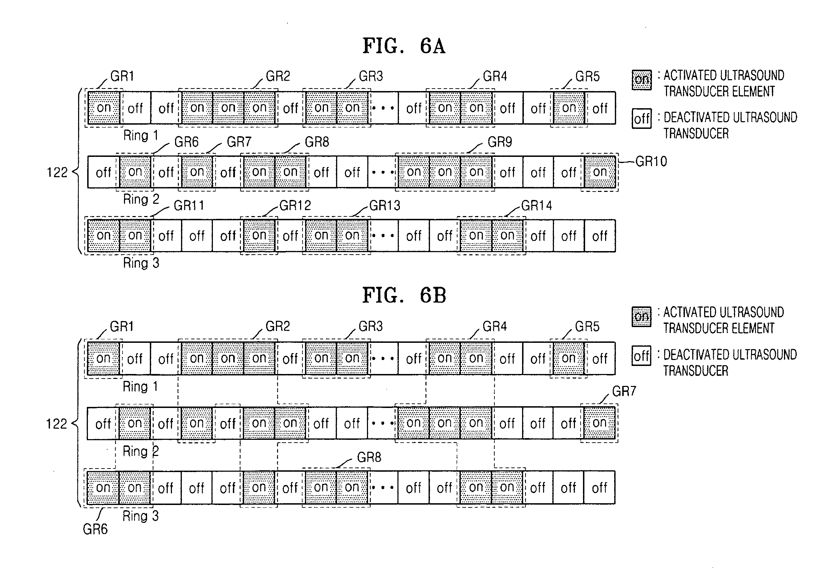

FIG. 6A and 6B are block diagrams illustrating an example of adjacent ultrasound transducer elements 122 grouped in an ultrasound transducer unit 120 having an array of ultrasound transducer elements 122 arranged in the form of a ring. That is, the example of the ultrasound transducer unit 120 illustrated in FIG. 2 has a structure in which a plurality of rings having different sizes, in which the ultrasound transducer elements 122 are arranged to form a circle in a lateral direction, are combined with each other in a vertical direction. FIGS. 6A and 6B each illustrate an array of ultrasound transducer elements 122 arranged in the form of three rings in the vertical directions.

The ultrasound transducer elements 122 of FIG. 6A and 6B have a sparse-array structure. The sparse-array structure refers to an arrangement in which some unsampled ultrasound transducer elements are deactivated and sampled ultrasound transducer elements are selected to perform signal processing. The grouping of the ultrasound transducer elements 122 are performed as a group of adjacent activated ultrasound transducer elements 122 in a single ring, as illustrated in the example of FIG. 6A, or as a group of adjacent ultrasound transducer elements 122 in adjacent rings, as illustrated in the example of FIG. 6B.

A distance between the adjacent activated ultrasound transducer elements is insignificantly less than a distance at which the adjacent activated ultrasound transducer elements reach a focus. Therefore, ultrasonic signals having the same phase may be transmitted onto the focus of a target region onto which ultrasound is to be irradiated, even though phases or amplitudes of ultrasound signals are the same. As a result, adjacent activated ultrasound transducer elements may be grouped into the same group.

Patterns of distribution of activated ultrasound transducer elements and deactivated ultrasound transducer elements are irregularly and randomly selected. This is because, when activated ultrasound transducer elements have a regular array arrangement, ultrasonic signals may be focused on other regions than the focus within the target region onto which ultrasound is to be irradiated.

In addition, a grouping unit of this example uses a genetic algorithm as an optimization technique to sample activated ultrasound transducer elements or group the activated ultrasound transducer elements. The genetic algorithm refers to a method of obtaining an optimum solution by using genetic manipulation and performance, which are obtained in an evolution procedure.

In accordance with the genetic algorithm, the grouping unit of this example generates a set of individuals. The individuals are samplings and groupings of activated ultrasound transducer elements. The grouping unit evaluates performance of the individuals to select one of the individuals to be meticulously evaluated. Here, the grouping unit generates a next generation set by performing manipulations, such as cross-linking, mutation, or any other manipulation known to one of ordinary skill in the art, on each of the individuals. As these manipulations are repeatedly performed, if the number of individuals having high performance increases by overlaying generations, the probability that an individual closest to an optimum solution may be generated increases.

For example, performance evaluation of the individuals may include an object function of a grating lobe region. Here, the grating lobe refers to a measurement of ultrasonic signals focused in other regions than the focus within the target region onto which ultrasound is to be irradiated. When the genetic algorithm is used, an individual that may minimize the grating lobe may be obtained as an optimum solution.

Referring again to the example illustrated in FIG. 9, after or simultaneously with the grouping of the ultrasound transducer elements, signals to be input according to groups are generated (20).

FIG. 11 is a flowchart illustrating an example of a generation of signals to be input according to groups in a method of controlling a medical apparatus. Referring to the example illustrated in FIG. 11, a signal generating unit generates (21) source signals.

The source signals are generated and inputted to a channel selection unit 412 via a plurality of input channels. For example, in FIG. 4, the source signals are generated and inputted to the channel selection unit 412 via a plurality of M channels SN_CH1 to SN_CHM.

The channel selection unit selects (22) output channels having a number of groups from among the input channels to output electrical signals according to groups of the plurality of ultrasound transducer elements. For example, in FIG. 4, the channel selection unit 412 selects output channels CH1 to CHN having the number of groups N from among the input channels SN_CH1 to SN_CHM to output electrical signals according to groups of the plurality of ultrasound transducer elements.

Referring to the example illustrated in FIGS. 4, 10, and 11, if the user inputs the number of groups N of the ultrasound transducer unit 420 to the user interface 430, input information regarding the number of groups N is outputted to the channel selection unit 412. Then, the channel selection unit 412 selects N channels CH1 to CHN according to the input information regarding the number of groups N. If the user does not input the number of groups N of the ultrasound transducer unit 420 to the user interface 430, the channel selection unit 412 is designed to select a predetermined number of channels according to predetermined settings.

Phases and amplitudes of the source signals input via the input channels, such as, for example, the plurality of M channels SN_CH1 to SN_CHM of FIG. 4, are modulated (23). Referring to the example illustrated in FIG. 7, the electrical signals generated by the signal generating unit 710 are input to the phase modulator 713, and phases of the electrical signals are modulated by phase modulator elements PT1 to PTN. In addition, amplifier elements AMP1 to AMPN of the signal amplifier 714 amplify the signals input from the phase modulator elements PT1 to PTN with appropriate gains, thereby outputting electrical signals via the output channels CH1 to CHN.

To this end, further referring to the example illustrated in FIG. 7, the grouping unit 740 transmits grouping information to the processor 760, and the processor 760 controls the phase modulator 713 and the signal amplifier 714, respectively, by analyzing the transmitted grouping information and calculating phases of electrical signals to be modulated by the phase modulator elements PT1 to PTN and calculating gains of the electrical signals to be amplified by the amplifier elements AMP1 to AMPN. In this way, different amplitudes and phases are applied to electrical signals input to the groups GP1 to GPN of the ultrasound transducer elements. Thus, the ultrasound signals generated from the ultrasound transducer elements that belong to different groups have different transmission delay times to compensate for the distance between the ultrasound transducer elements and the focus FC. Thus, in this example, the ultrasound irradiated by the plurality of ultrasound transducer elements is focused on the focus FC.

In addition, the phases and amplitudes of the electrical signals input to the groups of the ultrasound transducer unit 720 are controlled so that a position of the focus FC on which ultrasound is focused may vary with a predetermined target region that requires treatment or diagnosis, i.e., in a region onto which the ultrasound is to be irradiated. In addition, the phases and amplitudes of the electrical signals input to the groups of the ultrasound transducer unit 720 are controlled so that the ultrasound generated from the ultrasound transducer unit 720 may make a multi-focus.

Referring again to the example of FIG. 9, the signal generating unit inputs (30) the electrical signals to the plurality of output channels according to groups of the ultrasound transducer unit. In this regard, one or more of the output channels of the signal generating unit outputs a common signal to two or more ultrasound transducer elements. The ultrasound transducer unit generates (40) ultrasound signals from the electrical signals input according to groups. Ultrasound is irradiated (50) onto a particular part of the body of the person to be inspected that requires treatment or diagnosis of lesion. Accordingly, ultrasound images of lesions or a diagnosis of a disease to be removed is generated.

When the ultrasound signals are used in generating ultrasound images, ultrasound requires an intensity that is much less than intensity required in ultrasound used to remove lesions. Ultrasounds that are focused on the irradiated part and are partially reflected from or diffracted at tissue layers may be transformed into electrical pulses by using a probe and used in capturing images of tissues of the body. That is, with reference to the example illustrated in FIG. 8, the ultrasound signals that are reflected from or diffracted at the body may vibrate the piezoelectric transducer of the receiving probe 851, and the piezoelectric transducer may output electrical pulses having predetermined amplitudes and phases according to the vibrations. The electrical pulses output from the receiving probe 851 are input to the image processor 852 and are processed so that image data is generated. The image generating unit 853 generates ultrasound images based on the image data. In addition, the image generating unit 853 transmits the generated ultrasound images to the display unit 854, and the ultrasound images are displayed in graphics on the display unit 854.

The units described herein may be implemented using hardware components and software components, such as microphones, amplifiers, band-pass filters, audio to digital convertors, processing devices any other hardware or software components that one of ordinary skill in the art would deem applicable. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors. As used herein, a processing device configured to implement a function A includes a processor programmed to run specific software. In addition, a processing device configured to implement a function A, a function B, and a function C may include configurations, such as, for example, a processor configured to implement both functions A, B, and C, a first processor configured to implement function A, and a second processor configured to implement functions B and C, a first processor to implement function A, a second processor configured to implement function B, and a third processor configured to implement function C, a first processor configured to implement function A, and a second processor configured to implement functions B and C, a first processor configured to implement functions A, B, C, and a second processor configured to implement functions A, B, and C, and so on.

The software may include a computer program, a piece of code, an instruction, or some combination thereof, for independently or collectively instructing or configuring the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. In particular, the software and data may be stored by one or more computer readable recording mediums. The computer readable recording medium may include any data storage device that can store data which can be thereafter read by a computer system or processing device. Examples of the computer readable recording medium include read-only memory (ROM), random-access memory (RAM), CD-ROMs, magnetic tapes, floppy disks, optical data storage devices.

Program instructions to perform a method described herein, or one or more operations thereof, may be recorded, stored, or fixed in one or more computer-readable storage media. The program instructions may be implemented by a computer. For example, the computer may cause a processor to execute the program instructions. The media may include, alone or in combination with the program instructions, data files, data structures, and the like. Examples of computer-readable storage media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The program instructions, that is, software, may be distributed over network coupled computer systems so that the software is stored and executed in a distributed fashion. For example, the software and data may be stored by one or more computer readable storage mediums.

In addition, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein. In addition, the described unit to perform an operation or a method may be hardware, software, or some combination of hardware and software. For example, the unit may be a software package running on a computer or the computer on which that software is running.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A medical apparatus (100, 300, 400, 700, 800), comprising:
an ultrasound transducer unit (120, 320, 420, 720, 820) comprising a plurality of ultrasound transducer elements (122, 322, 522), the ultrasound transducer elements (122, 322, 522) being configured to transform signals into ultrasound and output the transformed ultrasound;
a grouping unit (140, 340, 400, 540, 740, 840) configured to group the ultrasound transducer elements (122, 322, 522) into a plurality of groups (GR1,...,GRN); and
a signal generating unit (110, 310, 410, 710, 810) configured to generate the signals to be transformed into the ultrasound and output the generated signals to the groups (GR1,...,GRN) via a plurality of output channels (CH1,...,CHN) respectively corresponding to the groups (GR1,...,GRN); **characterised in that**
the grouping unit (140, 340, 400, 540, 740, 840) is further configured to group the ultrasound transducer elements (122, 322, 522) into activated ultrasound transducer elements (122, 322, 522) and deactivated ultrasound transducer elements (122, 322, 522) via random extraction, and group the activated ultrasound transducer elements (122, 322, 522) into two or more groups (GR1,...,GRN).

2. The medical apparatus (100, 300, 400, 700, 800) according to claim 1, wherein the signal generating unit (110, 310, 410, 710, 810) is further configured to output one or more common signals of the signals to be transformed into the ultrasound via one or more of the output channels (CH1,...,CHN), respectively, to respective ones of the groups (GR1,...,GRN) comprising two or more of the ultrasound transducer elements (122, 322, 522).

3. The medical apparatus (100, 300, 400, 700, 800) according to any one of the preceding claims, further comprising:
a channel selection unit (412) configured to select two or more of the output channels (CH1,...,CHN) via which the signals to be transformed into the ultrasound are to be output to the groups (GR1,...,GRN);
a user interface (430, 730, 830) configured to receive grouping information specifying a quantity of the groups (GR1,...,GRN) in which the ultrasound transducer elements (122, 322, 522) are to be grouped,
wherein the channel selection unit (412) is further configured to select the two or more of the output channels (CH1,...,CHN) according to the received grouping information.

4. The medical apparatus (100, 300, 400, 700, 800) of claim 1, further comprising:
a user interface (430, 730, 830) configured to receive grouping information specifying a quantity of the activated ultrasound transducer elements (122, 322, 522) to be input,
wherein the grouping unit (140, 340, 400, 540, 740, 840) is further configured to group the activated ultrasound transducer elements (122, 322, 522) and deactivated ultrasound transducer elements (122, 322, 522) according to the received grouping information.

5. The medical apparatus (100, 300, 400, 700, 800) according to any one of the preceding claims, wherein the grouping unit (140, 340, 400, 540, 740, 840) is further configured to group adjacent ones of the ultrasound transducer elements (122, 322, 522) into a single group.

6. The medical apparatus (100, 300, 400, 700, 800) according to any one of the preceding claims, further comprising:
a phase modulator (713) configured to modulate phase of the signals to be outputted to the groups (GR1,...,GRN) to form a focus of the ultrasound transducer unit (120, 320, 420, 720, 820) in a target region onto which the ultrasound is to be irradiated;
a signal amplifier (714) configured to amplify amplitudes of the phase modulated signals;.
a processor (760) configured to calculate phases and amplification gains of the signals to be outputted to the groups (GR1,...,GRN) to control the phase modulator (713) and the signal amplifier (714), the phases and the amplification gains being calculated according to grouping information transmitted from the grouping unit (140, 340, 400, 540, 740, 840).

7. The medical apparatus (100, 300, 400, 700, 800) according to anyone of the preceding claims, wherein the processor (760) is further configured to control the phase modulator (713) and the signal amplifier (714) to form a multi-focus from the ultrasound irradiated by the ultrasound transducer unit (120, 320, 420, 720, 820) in response to the signals outputted to the groups (GR1,...,GRN).

## Patentansprüche

1. Medizinische Vorrichtung (100, 300, 400, 700, 800), umfassend:
eine Ultraschallwandler-Einheit (120, 320, 420, 720, 820), die eine Vielzahl von Ultraschallwandler-Elementen (122, 322, 522) umfasst, wobei die Ultraschallwandler-Elemente (122, 322, 522) konfiguriert sind, Signale in Ultraschall umzuwandeln und den umgewandelten Ultraschall auszugeben;
eine Gruppierungseinheit (140, 340, 400, 540, 740, 840), die konfiguriert ist, die Ultraschallwandler-Elemente (122, 322, 522) in eine Vielzahl von Gruppen (GR1,...,GRN) zu gruppieren; und
eine Signalerzeugungseinheit (110, 310, 410, 710, 810), die konfiguriert ist, die Signale zu erzeugen, die in den Ultraschall umgewandelt werden sollen, und die erzeugten Signal an die Gruppen (GR1,...,GRN) über eine Vielzahl von Ausgabekanälen (CH1,...,CHN), die jeweils den Gruppen (GR1,...,GRN) entsprechen, auszugeben;
**dadurch gekennzeichnet, dass**
die Gruppierungseinheit (140, 340, 400, 540, 740, 840) weiterhin konfiguriert ist, die Ultraschallwandler-Elemente (122, 322, 522) in aktivierte Ultraschallwandler-Elemente (122, 322, 522) und deaktivierte Ultraschallwandler-Elemente (122, 322, 522) mittels zufälliger Auswahl zu gruppieren, und die aktivierten Ultraschallwandler-Elemente (122, 322, 522) in zwei oder mehr Gruppen (GR1,...,GRN) zu gruppieren.

2. Medizinische Vorrichtung (100, 300, 400, 700, 800) gemäß Anspruch 1, wobei die Signalerzeugungseinheit (110, 310, 410, 710, 810) weiterhin konfiguriert ist, eines oder mehrere gemeinsame Signale aus den Signalen, die über die ein oder mehreren Ausgabekanäle (CH1,...,CHN) jeweils in den Ultraschall umgewandelt werden sollen, jeweils an die entsprechenden der Gruppen (GR1,...,GRN) auszugeben, die zwei oder mehrere der Ultraschallwandler-Elemente (122, 322, 522) umfassen.

3. Medizinische Vorrichtung (100, 300, 400, 700, 800) gemäß wenigstens einem der vorherigen Ansprüche, weiterhin umfassend:
eine Kanalauswahleinheit (412), die konfiguriert ist, zwei oder mehrere der Ausgabekanäle (CH1,...,CHN), über die die Signale, die in den Ultraschall umgewandelt werden sollen, an die Gruppen (GR1,...,GRN) ausgegeben werden sollen, auszuwählen;
eine Benutzerschnittstelle (430, 730, 830), die konfiguriert ist, Gruppierungsinformation zu empfangen, die eine Anzahl aus den Gruppen (GR1,...,GRN) spezifiziert, in welche die Ultraschallwandler-Elemente (122, 322, 522) gruppiert werden sollen,
wobei die Kanalauswahleinheit (412) weiterhin konfiguriert ist, zwei oder mehrere der Ausgabekanäle(CH1,...,CHN) entsprechend der empfangenen Gruppierungsinformation auszuwählen.

4. Medizinische Vorrichtung (100, 300, 400, 700, 800) gemäß Anspruch 1, weiterhin umfassend:
eine Benutzerschnittstelle (430, 730, 830), die konfiguriert ist, Gruppierungsinformation zu empfangen, die eine Anzahl von den aktivierten Ultraschallwandler-Elementen (122, 322, 522) spezifiziert, die eingegeben werden soll,
wobei die Gruppierungseinheit (140, 340, 400, 540, 740, 840) weiterhin konfiguriert ist, die aktivierten Ultraschallwandler-Elemente (122, 322, 522) und die deaktivierten Ultraschallwandler-Elemente (122, 322, 522) entsprechend der empfangenen Gruppierungsinformation zu gruppieren.

5. Medizinische Vorrichtung (100, 300, 400, 700, 800) gemäß wenigstens einem der vorherigen Ansprüche, wobei die Gruppierungseinheit (140, 340, 400, 540, 740, 840) weiterhin konfiguriert ist, benachbarte der Ultraschallwandler-Elemente (122, 322, 522) in eine einzige Gruppe zu gruppieren.

6. Medizinische Vorrichtung (100, 300, 400, 700, 800) gemäß wenigstens einem der vorherigen Ansprüche, weiterhin umfassend:
einen Phasenmodulator (713), der konfiguriert ist, eine Phase der Signale, die an die Gruppen (GR1,...,GRN) ausgegeben werden sollen, zu modulieren, um einen Fokus der Ultraschallwandler-Einheit (120, 320, 420, 720, 820) in einem Zielbereich zu formen, auf den der Ultraschall eingestrahlt werden soll;
einen Signalverstärker (714), der konfiguriert ist, die Amplitude der phasenmodulierten Signale zu verstärken;
einen Prozessor (760), der konfiguriert ist, Phasen und Verstärkungen der Signale, die an die Gruppen (GR1,...,GRN) ausgegeben werden sollen, zu berechnen, um den Phasenmodulator (713) und den Signalverstärker (714) zu steuern, wobei die Phasen und die Verstärkungen berechnet werden entsprechend von Gruppierungsinformation, die von der Gruppierungseinheit (140, 340, 400, 540, 740, 840) gesendet wird.

7. Medizinische Vorrichtung (100, 300, 400, 700, 800) gemäß wenigstens einem der vorherigen Ansprüche, wobei der Prozessor (760) weiterhin konfiguriert ist, den Phasenmodulator (713) und den Signalverstärker (714) zu steuern, um einen Multifokus aus dem Ultraschall, der von der Ultraschallwandler-Einheit (120, 320, 420, 720, 820) in Antwort auf die Signale, die an die Gruppen (GR1,...,GRN) ausgegeben werden, zu formen.

## Revendications

1. Appareil médical (100, 300, 400, 700, 800), comprenant :
une unité transducteur à ultrason (120, 320, 420, 720, 820) comprenant une pluralité d'éléments transducteurs à ultrason (122, 322, 522), les éléments transducteurs à ultrason (122, 322, 522) étant configurés pour transformer des signaux en ultrason et pour sortir l'ultrason transformé ;
une unité de groupement (140, 340, 400, 540, 740, 840) configurée pour grouper les éléments transducteurs à ultrason (122, 322, 522) en une pluralité de groupes (GR1,...,GRN) ; et
une unité de génération de signaux (110, 310, 410, 710, 810) configurée pour générer les signaux à transformer en ultrason et pour sortir les signaux générés vers les groupes (GR1,...,GRN) via une pluralité de canaux de sortie (CH1,...,CHN) correspondant respectivement aux groupes (GR1,...,GRN) ;
**caractérisé en ce que**
l'unité de groupement (140, 340, 400, 540, 740, 840) est en outre configurée pour grouper les éléments transducteurs à ultrason (122, 322, 522) en éléments transducteurs à ultrason activés (122, 322, 522) et en éléments transducteurs à ultrason désactivés (122, 322, 522) via une extraction aléatoire, et pour grouper les éléments transducteurs à ultrason activés (122, 322, 522) en deux ou plusieurs groupes (GR1,...,GRN).

2. Appareil médical (100, 300, 400, 700, 800) selon la revendication 1, dans lequel l'unité de génération de signaux (110, 310, 410, 710, 810) est en outre configurée pour sortir un ou plusieurs signaux communs des signaux à transformer en ultrason via respectivement un ou plusieurs des canaux de sortie (CH1,...,CHN) vers les groupes respectifs (GR1,...,GRN) comprenant deux ou plusieurs des éléments transducteurs à ultrason (122, 322, 522).

3. Appareil médical (100, 300, 400, 700, 800) selon l'une quelconque des revendications précédentes, comprenant en outre :
une unité de sélection de canal (412) configurée pour sélectionner deux ou plusieurs des canaux de sortie (CH1,...,CHN) via lesquels les signaux à transformer en ultrason doivent être sortis vers les groupes (GR1,...,GRN) ;
une interface d'utilisateur (430, 730, 830) configurée pour recevoir de l'information de groupement qui spécifie une quantité des groupes (GR1,...,GRN) dans lesquels les éléments transducteurs à ultrason (122, 322, 522) doivent être groupés,
dans lequel l'unité de sélection de canal (412) est en outre configurée pour sélectionner deux ou plusieurs des canaux de sortie (CH1,...,CHN) en fonction de l'information de groupement reçue.

4. Appareil médical (100, 300, 400, 700, 800) selon la revendication 1, comprenant en outre :
une interface d'utilisateur (430, 730, 830) configurée pour recevoir de l'information de groupement qui spécifie une quantité des éléments transducteurs à ultrason activés (122, 322, 522) à entrer,
dans lequel l'unité de groupement (140, 340, 400, 540, 740, 840) est en outre configurée pour grouper les éléments transducteurs à ultrason activés (122, 322, 522) et les éléments transducteurs à ultrason désactivés (122, 322, 522) en fonction de l'information de groupement reçue.

5. Appareil médical (100, 300, 400, 700, 800) selon l'une quelconque des revendications précédentes, dans lequel l'unité de groupement (140, 340, 400, 540, 740, 840) est en outre configurée pour grouper des éléments adjacents parmi les éléments transducteurs à ultrason (122, 322, 522) en un seul groupe.

6. Appareil médical (100, 300, 400, 700, 800) selon l'une quelconque des revendications précédentes, comprenant en outre :
un modulateur de phase (713) configuré pour moduler la phase des signaux à sortir vers les groupes (GR1,...,GRN) pour former un foyer de l'unité transducteur à ultrason (120, 320, 420, 720, 820) dans une zone cible sur laquelle l'ultrason doit être irradié ;
un amplificateur de signal (714) configuré pour amplifier les amplitudes des signaux modulés en phase ;
un processeur (760) configuré pour calculer des phases et des gains d'amplification des signaux à sortir vers les groupes (GR1,...,GRN) pour contrôler le modulateur de phase (713) et l'amplificateur de signal (714), les phases et les gains d'amplification étant calculés en fonction d'information de groupement transmise à partir de l'unité de groupement (140, 340, 400, 540, 740, 840).

7. Appareil médical (100, 300, 400, 700, 800) selon l'une quelconque des revendications précédentes, dans lequel le processeur (760) est en outre configuré pour contrôler le modulateur de phase (713) et l'amplificateur de signal (714) pour former un foyer multiple à partir de l'ultrason irradié par l'unité transducteur à ultrason (120, 320, 420, 720, 820) en réponse aux signaux sortis vers les groupes (GR1,...,GRN).
